# EUROPEAN PATENT APPLICATION

(11) **EP 3 677 912 A1**
(43) Date of publication of application: **08.07.2020**
(21) Application number: 18849626.9
(22) Date of filing: 12.07.2018
(51) Int. Cl.: G01N 33/576, G01N 21/41, G01N 33/543, G01N 33/553, G01N 33/58

(54) **METHOD AND KIT FOR DETECTING HEPATITIS B SURFACE ANTIGEN**

(30) Priority: 31.08.2017 JP 2017167618
(71) Applicant: Konica Minolta, Inc., Tokyo 100-7015 (JP)
(72) Inventor: TERADA, Kotaro, Tokyo 100-7015 Japan (JP)
(74) Representative: Gille Hrabal
(86) International application number: PCT/JP2018/026280
(87) International publication number: WO 2019/044202

(57) **Abstract**

The present invention pertains to a method for detecting HBsAg with which it is possible to detect HBsAg with high sensitivity even when blood (whole blood) is used as a sample. A sample is provided on a metal film on the surface of which there is immobilized a binding substance (e.g., an antibody) capable of specifically binding to hepatitis B surface antigens, and hepatitis B surface antigens included in the sample are bound by the binding substance. The hepatitis B surface antigens are also labeled by a fluorescent substance. Fluorescence, which is emitted from the fluorescent substance when the metal film is irradiated with excitation light so that surface plasmon resonance is produced in the metal film, is detected.

## Description

### Technical Field

The present invention relates to a detection method and a detection kit for a hepatitis B virus surface antigen.

### Background Art

Hepatitis B is a viral hepatitis caused by infection with hepatitis B virus (hereinafter also referred to as "HBV"). A hepatitis B virus surface antigen (hereinafter also referred to as "HBsAg") corresponding to an envelope antigen of HBV is released into the blood when HBV grows in hepatocytes. Therefore, infection with HBV can be examined by detecting an HBsAg in the blood. An HBsAg is transiently detected in the blood in the case of transient HBV infection, and is persistently detected in the blood in the case of persistent HBV infection.

In order to highly precisely examine HBV infection, it is necessary to highly sensitively detect an HBsAg. For example, PTL 1 discloses that an HBsAg in serum can be highly sensitively detected by using a combination of an antibody binding to the S region of an HBsAg and an antibody binding to the Pre-S1 region and Pre-S2 region.

### Citation List

### Patent Literatures

PTL 1: Japanese Patent Application Laid-Open No. 2001-133460

### Summary of Invention

### Technical Problem

In order to highly sensitively detect an HBsAg, while prescription of a reagent to be used may be devised as in the invention described in PTL 1, a detection method having high detection sensitivity in principle may be employed. For simply performing examination, a detection method in which not serum or plasma but blood (whole blood) can be directly used as a specimen is preferred. However, a detection method by which an HBsAg can be highly sensitively detected by using blood (whole blood) as a specimen has not been proposed until now.

The present invention was accomplished in consideration of these points, and an object is to provide a detection method and a detection kit for an HBsAg by which an HBsAg can be highly sensitively detected even when blood (whole blood) is used as a specimen.

### Solution to Problem

A detection method for an HBsAg, according to one embodiment of the present invention comprises: preparing a detection chip including a metal film and a binding substance which is immobilized on the metal film and which specifically binds to an HBsAg; providing a specimen onto the metal film to cause an HBsAg contained in the specimen to bind to the binding substance; labeling, with a fluorescent substance, the HBsAg before or after binding to the binding substance; and detecting fluorescence emitted from the fluorescent substance when the metal film is irradiated with excitation light in such a manner as to generate surface plasmon resonance in the metal film with the HBsAg labeled with the fluorescent substance kept in a state binding to the binding substance.

Furthermore, a detection kit for an HBsAg, according to one embodiment of the present invention comprises: a detection chip including a metal film and a binding substance which is immobilized on the metal film and which specifically binds to an HBsAg; and a labeling reagent for labeling am HBsAg with a fluorescent substance.

### Advantageous Effects of Invention

According to the present invention, an HBsAg can be highly sensitively detected even when blood (whole blood) is used as a specimen.

### Brief Description of Drawings

[FIG. 1] FIG. 1 is a flowchart illustrating an example of a detection method for an HBsAg according to the present embodiment.
[FIG. 2] FIG. 2A is a schematic cross-sectional view illustrating a structure of a detection chip for use in PC-SPFS, and FIG. 2B is a schematic cross-sectional view illustrating a structure of a detection chip for use in GC-SPFS.
[FIG. 3] FIG. 3 is a schematic cross-sectional view illustrating an example of a detection chip for use in PC-SPFS.
[FIG. 4] FIG. 4 is a graph illustrating test results for dilution linearity.
[FIG. 5] FIG. 5 is a graph illustrating the relationship between detection results obtained by the detection method of the present embodiment and detection results obtained by another detection method.

### Description of Embodiments

Embodiments of the present invention will now be described in detail with reference to the accompanying drawings.

### [Detection Method for HBsAg]

In a detection method for an HBsAg of the present embodiment, an HBsAg is detected by utilizing surface plasmon-field enhanced fluorescence spectroscopy (hereinafter also referred to as "SPFS"). In SPFS, a fluorescent substance is excited to emit fluorescence by an electric field enhanced by surface plasmon resonance (hereinafter also referred to as "SPR"), and therefore, as compared with general fluoroimmunoassay, a target (that is, an HBsAg in the present embodiment) can be highly sensitively detected. In SPFS, whole blood can be used as a specimen.

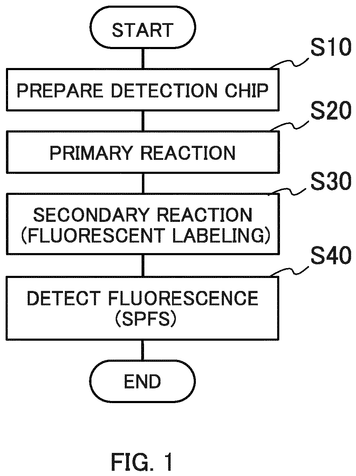

The detection method for an HBsAg of the present embodiment will now be specifically described. FIG. 1 is a flowchart illustrating an example of the detection method for an HBsAg of the present embodiment.

### (Preparation of Detection Chip)

First, a detection chip including a metal film and a binding substance specifically binding to an HBsAg is prepared (step S10). In SPFS, SPR is generated by causing evanescent waves, caused by irradiating a metal film with light (that is, excitation light in the present embodiment), and surface plasmon to couple to each other. As a method for generating SPR, a method in which a prism is disposed on one plane of a metal film (Kretschmann configuration), a method in which a diffraction grating is formed in a metal film, and the like are known. SPFS employing the former method is designated as prism coupling (PC)-SPFS, and SPFS employing the latter method is designated as grating coupling (GC)-SPFS. The detection method for an HBsAg of the present embodiment may employ either of PC-SPFS and GC-SPFS.

As described above, when a metal film is irradiated with excitation light, SPR is generated. The type of a metal constituting the metal film is not particularly limited as long as the metal can generate SPR. Examples of the metal constituting the metal film include gold, silver, copper, aluminum and an alloy thereof.

The binding substance can specifically bind to an HBsAg, and is immobilized on the metal film for capturing an HBsAg contained in a specimen. In general, the binding substance is uniformly immobilized in a prescribed region (reaction field) on the metal film. The type of the binding substance immobilized on the metal film is not particularly limited as long as it can specifically bind to an HBsAg. Examples of the binding substance include an antibody capable of specifically binding to an HBsAg (an anti-HBsAg antibody), a nucleic acid capable of specifically binding to an HBsAg, a lipid capable of specifically binding to an HBsAg, and a protein, excluding an antibody, capable of specifically binding to an HBsAg. When the binding substance is an anti-HBsAg antibody, the anti-HBsAg antibody may be a monoclonal antibody, a polyclonal antibody, or a fragment of an antibody. One or two or more binding substances may be immobilized on the metal film. For example, the anti-HBsAg antibody immobilized on the metal film may be one or two or more anti-hepatitis B virus surface antigen monoclonal antibodies or anti-hepatitis B virus surface antigen polyclonal antibodies.

From the viewpoint of improving detection sensitivity for an HBsAg, an antibody specifically binding to a specific region of the HBsAg may be used as the binding substance immobilized on the metal film. For example, the anti-HBsAg antibody immobilized on the metal film may be an antibody binding to the S region of an HBsAg, or may be a combination of an antibody binding to the S region of an HBsAg and an antibody binding to the Pre-S2 region of an HBsAg.

A method for immobilizing the binding substance is not particularly limited. For example, a self-assembled monolayer (hereinafter referred to as "SAM") or a polymer film to which the binding substance (such as an anti-HBsAg antibody) is caused to bind may be formed on the metal film. An example of the SAM includes a film made of a substituted aliphatic thiol such as HOOC-(CH₂)₁₁-SH. Examples of a material constituting the polymer film include polyethylene glycol and MPC polymer. A polymer having a reactive group (or a functional group that can be converted into a reactive group) capable of binding to the binding substance (such as an anti-HBsAg antibody) may be immobilized on the metal film, and the binding substance (such as an anti-HBsAg antibody) may be caused to bind to the polymer.

A detection chip is a structure having each side with a length preferably of several mm to several cm, and may be a more compact structure or a larger structure not belonging to the category of "chip".

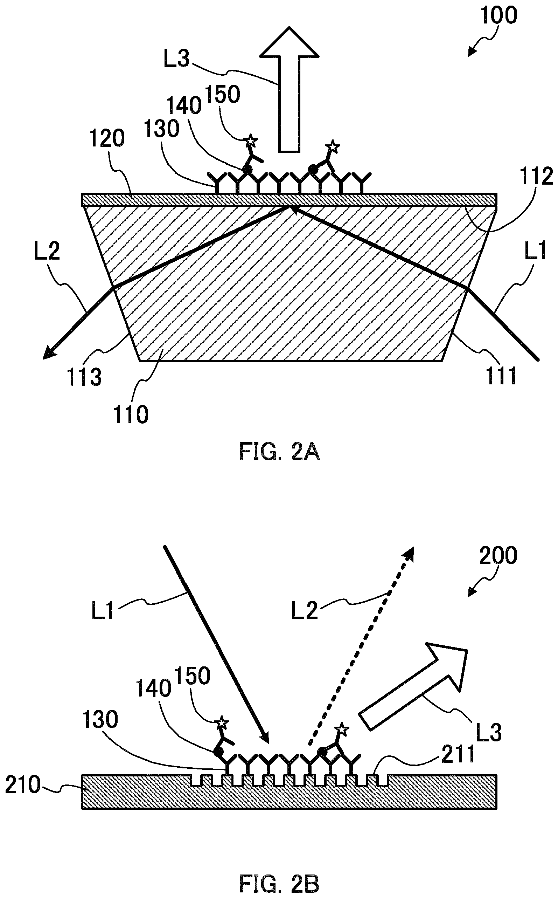

FIG. 2A is a schematic cross-sectional view illustrating the structure of a detection chip for use in PC-SPFS, and FIG. 2B is a schematic cross-sectional view illustrating the structure of a detection chip for use in GC-SPFS. For convenience of description, the sizes and the shapes of the respective constituent elements are not accurate in these drawings. These drawings show examples in which an anti-HBsAg antibody is used as the binding substance.

As illustrated in FIG. 2A, detection chip 100 for use in PC-SPFS includes prism 110, metal film 120 and (a layer of) anti-HBsAg antibody 130. Prism 110 is made of a dielectric transparent to excitation light L1, and includes entrance surface 111 where excitation light L1 enters, film surface 112 on which excitation light L1 reflects, and exit surface 113 through which reflected light L2 exits. The shape of prism 110 is not particularly limited. In the exemplified case of FIG. 2A, prism 110 is in a column shape having a trapezoidal bottom. A surface corresponding to one base of the trapezoid is film surface 112, a surface corresponding to one leg is entrance surface 111, and a surface corresponding to the other leg is exit surface 113. Examples of a material of prism 110 include a resin and glass. The material of prism 110 is preferably a resin having a refractive index for excitation light of 1.4 to 1.6 and having low birefringence. Metal film 120 is disposed on film surface 112 of prism 110. The method for forming metal film 120 is not particularly limited. Examples of the method for forming metal film 120 include sputtering, deposition and plating. A thickness of metal film 120 is not particularly limited, and is preferably in a range of 30 to 70 nm.

As illustrated in FIG. 2A, when metal film 120 is irradiated with excitation light L1 through prism 110 so as to generate SPR in metal film 120, an electric field enhanced by SPR is generated in the vicinity of metal film 120. At this point, when HBsAg 140 labeled with fluorescent substance 150 binds to anti-HBsAg antibody 130 disposed on metal film 120, fluorescent substance 150 is excited by the enhanced electric field to emit fluorescence L3.

As illustrated in FIG. 2B, detection chip 200 for use in GC-SPFS includes metal film 210 having diffraction grating 211 formed therein and (a layer of) anti-HBsAg antibody 130. The method for forming metal film 210 is not particularly limited. Examples of the method for forming metal film 210 include sputtering, deposition and plating. The thickness of metal film 210 is not particularly limited, and is preferably in a range of 30 to 500 nm. The shape of diffraction grating 211 is not particularly limited as long as evanescent waves can be caused. For example, diffraction grating 211 may be a one-dimensional diffraction grating or a two-dimensional diffraction grating. For example, as a one-dimensional diffraction grating, a plurality of convexities parallel to one another are formed at prescribed intervals on the surface of metal film 210. As a two-dimensional diffraction grating, projections in a prescribed shape are periodically arranged on the surface of metal film 210. Examples of arrangement of the projections include square lattice arrangement and triangular (hexagonal) lattice arrangement. Examples of a cross-sectional shape of diffraction grating 211 include a square wave shape, a sine wave shape and a sawtooth shape. A method for forming diffraction grating 211 is not particularly limited. For example, metal film 210 may be provided with a concave/convex shape after being formed on a plate-shaped substrate (not shown). Alternatively, metal film 210 may be formed on a substrate (not shown) precedently provided with a concave/convex shape. No matter which method is employed, metal film 210 including diffraction grating 211 can be formed.

As illustrated in FIG. 2B, when metal film 210 (diffraction grating 211) is irradiated with excitation light L1 so as to generate SPR in metal film 210 (diffraction grating 211), an electric field enhanced by SPR is generated in the vicinity of metal film 210 (diffraction grating 211). At this point, when HBsAg 140 labeled with fluorescent substance 150 binds to anti-HBsAg antibody 130 disposed on metal film 210 (diffraction grating 211), fluorescent substance 150 is excited by the enhanced electric field to emit fluorescence L3.

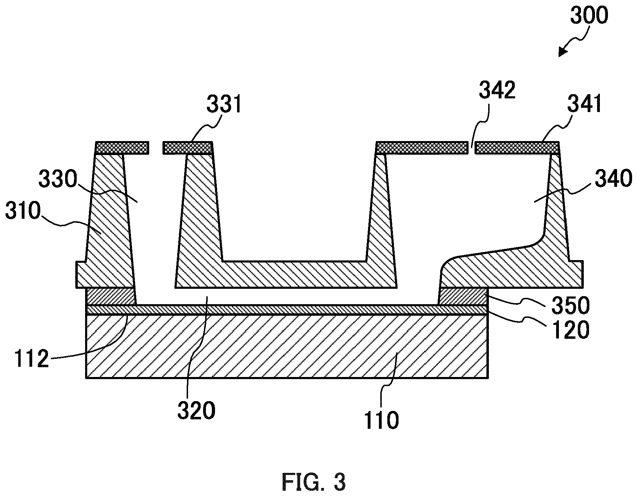

FIG. 3 is a schematic cross-sectional view illustrating an example of the detection chip for use in PC-SPFS. As illustrated in FIG. 3, detection chip 300 includes prism 110 having entrance surface 111, film surface 112 and exit surface 113, metal film 120 formed on film surface 112 of prism 110, and passage cover 310 disposed on film surface 112 of prism 110 or metal film 120. In FIG. 3, entrance surface 111 and exit surface 113 are present respectively in front of and behind a sheet surface of the drawing. Detection chip 300 further includes passage 320, liquid injection port 330 connected to one end of passage 320, and reservoir 340 connected to the other end of passage 320. In the present embodiment, passage cover 310 is caused to adhere to metal film 120 (or prism 110) through adhesive layer 350 of a double sided tape or the like, and adhesive layer 350 also plays a role to define the shape of a side surface of passage 320. Although not illustrated in FIG. 3, anti-HBsAg antibody 130 is immobilized in a partial region (reaction field) of metal film 120 exposed in passage 320. Liquid injection port 330 is closed by liquid injection port covering film 331, and reservoir 340 is closed by reservoir covering film 341. Reservoir covering film 341 is provided with vent 342.

Passage cover 310 is made of a material transparent to fluorescence L3. It is noted that a part of passage cover 310 may be made of a material not transparent to fluorescence L3 as long as outcoupling of fluorescence L3 cannot be prevented. An example of the material transparent to fluorescence L3 includes a resin. Passage cover 310 may be connected, without using adhesive layer 350, to metal film 120 (or prism 110) through laser welding, ultrasonic welding, pressure bonding with a clamp member or the like. In this case, the shape of the side surface of passage 320 is defined by passage cover 310.

A pipette tip is inserted into liquid injection port 330. At this point, an opening of liquid injection port 330 (that is, a through hole provided in liquid injection port covering film 331) comes into tight contact with the outer circumference of the pipette tip. Therefore, a liquid can be introduced into passage 320 by injecting the liquid into liquid injection port 330 from the pipette tip, and a liquid held in passage 320 can be removed by sucking the liquid held in liquid injection port 330 into the pipette tip. When injection and suction of a liquid are alternately performed, the liquid can be fed to reciprocate in passage 320.

When a liquid in an amount exceeding the volume of passage 320 is introduced from liquid injection port 330 into passage 320, the liquid flows from passage 320 into reservoir 340. Also when a liquid is fed to reciprocate in passage 320, the liquid flows into reservoir 340. The liquid thus flown into reservoir 340 is stirred within reservoir 340. When the liquid is stirred in reservoir 340, a concentration of a component (such as an HBsAg or a cleaning component) of the liquid (such as a specimen or a cleaning liquid) passing through passage 320 is made uniform, and hence various reactions can be easily caused in passage 320, or a cleaning effect is improved.

### (Primary Reaction)

Next, a specimen is provided onto the metal film of the detection chip to cause an HBsAg contained in the specimen to bind to the binding substance (primary reaction; step S20). A method for providing a specimen is not particularly limited. For example, a specimen may be provided onto the metal film using a pipette having a pipette tip attached to a tip thereof. In general, after completing the primary reaction, the surface of the metal film is cleaned with a buffer or the like to remove a component not binding to the binding substance.

The type of the specimen is not particularly limited. Examples of the specimen include blood, serum, plasma, and a diluted solution thereof. In the detection method for an HBsAg of the present embodiment, an HBsAg is detected by employing SPFS, and hence, whole blood can be used as the specimen.

### (Secondary Reaction)

Next, a labeling reagent is provided onto the metal film of the detection chip to label, with a fluorescent substance, the HBsAg having bound to the binding substance (secondary reaction; step S30). A method for providing a labeling reagent is not particularly limited. For example, a labeling reagent may be provided onto the metal film using a pipette having a pipette tip attached to a tip thereof. In general, after completing the secondary reaction, the surface of the metal film is cleaned with a buffer or the like to remove the fluorescent substance not labeling the HBsAg.

The type of the labeling reagent is not particularly limited as long as the HBsAg having bound to the binding substance can be labeled with a fluorescent substance. For example, the labeling reagent is a binding substance, labeled with a fluorescent substance, specifically binding to an HBsAg. The type of the binding substance contained in the labeling reagent is not particularly limited as long as it can specifically bind to an HBsAg. Examples of the binding substance include an antibody capable of specifically binding to an HBsAg (an anti-HBsAg antibody), a nucleic acid capable of specifically binding to an HBsAg, a lipid capable of specifically binding to an HBsAg, and a protein, excluding an antibody, capable of specifically binding to an HBsAg. The binding substance contained in the labeling reagent may be the same type as or different type from the binding substance immobilized on the metal film. When the binding substance is an anti-HBsAg antibody, the anti-HBsAg antibody may be a monoclonal antibody, a polyclonal antibody, or a fragment of an antibody. One or two or more binding substances may be immobilized on the metal film. For example, the anti-HBsAg antibody labeled with a fluorescent substance may be one or two or more anti-HBsAg monoclonal antibodies or anti-HBsAg polyclonal antibodies. In this case, the anti-HBsAg monoclonal antibodies and the anti-HBsAg polyclonal antibodies labeled with a fluorescent substance are preferably different from one or two or more anti-HBsAg monoclonal antibodies immobilized on the metal film.

From the viewpoint of improving detection sensitivity for an HBsAg, an antibody binding to a specific region of an HBsAg may be used as the anti-HBsAg antibody labeled with a fluorescent substance. For example, the anti-HBsAg antibody labeled with a fluorescent substance may be an antibody binding to the S region of an HBsAg, or may be a combination of an antibody binding to the S region of an HBsAg and an antibody binding to the Pre-S2 region of an HBsAg.

The type of the fluorescent substance is not particularly limited as long as it can be used in SPFS. Examples of the fluorescent substance include cyanine-based dyes, Alex Fluor(R) dye of Thermo Scientific, and CF dye of Biotium. Alexa Fluor dye and CF dye have high quantum efficiency for the wavelength of excitation light used in SPFS as compared with other commercially available fluorescent dyes. CF dye is not largely discolored in fluorescence detection, and hence the fluorescence detection can be stably performed. A method for labeling the binding substance with a fluorescent substance is not particularly limited, and can be appropriately selected from known methods. For example, a fluorescent substance may be caused to bind to an amino group or a sulfhydryl group of the binding substance (such as an anti-HBsAg antibody).

Although an HBsAg is labeled with the fluorescent substance after causing the HBsAg to bind to the binding substance immobilized on the metal film in the above description, an HBsAg may be labeled with the fluorescent substance before causing the HBsAg to bind to the binding substance immobilized on the metal film. In this case, the specimen and the labeling reagent may be mixed before providing the specimen onto the metal film. Alternatively, a step of causing the binding substance immobilized on the metal film to bind to an HBsAg and a step of labeling the HBsAg with the fluorescent substance may be simultaneously performed. In this case, the specimen and the labeling reagent may be simultaneously provided onto the metal film.

### (Fluorescence Detection)

Next, fluorescence corresponding to the presence or amount of the HBsAg is detected by SPFS (step S40). Specifically, with the HBsAg labeled with the fluorescent substance and binding to the binding substance immobilized on the metal film, the metal film is irradiated with the excitation light so as to generate SPR, and fluorescence thus emitted from the fluorescent substance is detected. In general, a precedently measured optical blank value is subtracted from a measured fluorescent value to calculate a signal value in correlation with the amount of the HBsAg. If necessary, the signal value may be converted into the amount or concentration of the HBsAg by using a calibration curve or the like precedently created.

When detection chip 100 for use in PC-SPFS is used, metal film 120 is irraciated with excitation light L1 through prism 110 as illustrated in FIG. 2A. Thus, SPR is generated in metal film 120, and fluorescent substance 150 present in the vicinity of metal film 120 is excited by the enhanced electric field to emit fluorescence L3. An incident angle of excitation light L1 against metal film 120 is set so as to generate SPR in metal film 120, and is preferably a resonance angle or a reinforcement angle. Here, the term "resonance angle" means an incident angle at which light intensity of reflected light L2 is minimum when the incident angle of excitation light L1 against metal film 120 is scanned. The term "reinforcement angle" means an incident angle at which light intensity of scattered light (plasmon scattered light) of the same wavelength as excitation light L1 emitted upward of metal film 120 (opposite to prism 110) is maximum when the incident angle of excitation light L1 against metal film 120 is scanned.

When detection chip 200 for use in GC-SPFS is used, metal film 210 (diffraction grating 211) is directly irradiated with excitation light L1 as illustrated in FIG. 2B. Thus, SPR is generated in metal film 210 (diffraction grating 211), and fluorescent substance 150 present in the vicinity of metal film 210 (diffraction grating 211) is excited by the enhanced electric field to emit fluorescence L3. An incident angle of excitation light L1 against metal film 210 is set so as to generate SPR in metal film 210, and is preferably an angle at which intensity of the enhanced electric field formed by SPR is maximum. An optimal incident angle of excitation light L1 is appropriately set in accordance with the pitch of diffraction grating 211, the wavelength of excitation light LI, the type of the metal constituting metal film 210 and the like.

The type of the excitation light is not particularly limited, and is generally laser light. For example, the excitation light is laser light emitted from a laser light source having an output power of 15 to 30 mW. When the output power is 15 mW or more, fluorescence intensity can be increased to appropriately detect the fluorescence. When the output power is 30 mW or less, the binding substance immobilized on the metal film and the like can be prevented from being harmfully affected. The wavelength of the excitation light is appropriately set in accordance with the excitation wavelength of the fluorescent substance to be used.

A detector for the fluorescence is preferably disposed, with respect to the detection chip, in a direction where the fluorescence intensity is the highest. For example, when detection chip 100 for use in PC-SPFS is used, the direction where the intensity of fluorescence L3 is the highest is a normal direction of metal film 120 as illustrated in FIG. 2A, and hence, the detector is disposed directly above the detection chip. On the other hand, when detection chip 200 for use in GC-SPFS is used, the direction where the intensity of fluorescence L3 is the highest is a direction somewhat inclined against the normal direction of metal film 120 as illustrated in FIG. 2B, and hence, the detector is disposed in a position not directly above the detection chip. The detector is, for example, a photomultiplier tube (PMT), an avalanche photodiode (APD) or the like.

Through the above-described procedures, the presence or amount of an HBsAg contained in a specimen can be detected.

### [Detection Kit for HBsAg]

A detection kit for an HBsAg according to the present embodiment is a set of the aforementioned detection chip and the aforementioned labeling reagent. When the detection chip and the labeling reagent are thus precedently prepared as a set, a user (such as a health care provider) can more simply perform the detection method for an HBsAg.

### [Effects]

As described so far, since SPFS is employed in the detection method or the detection kit for an HBsAg of the present embodiment, an HBsAg can be highly sensitively detected in a short period of time even when blood (whole blood) is used as a specimen.

### Examples

Now, the present invention will be described in detail with reference to Examples, and it is noted that the present invention is not limited to these Examples.

### Experiment 1: Comparison between Measured Value for Whole Blood and Measured Value for Plasma

Detection chip 300 having the structure illustrated in FIG. 3 was prepared. A mouse anti-HBsAg monoclonal antibody (Institute of Immunology Co., Ltd.) was immobilized in a specific region (reaction portion) of metal film 120 (metal film) exposed in passage 320.

Blood was collected from four healthy volunteers. 10 µL of purified HBsAg (Institute of Immunology Co., Ltd.) was added to 1990 µL of the blood (whole blood) of each of these four volunteers. The thus obtained blood to which the antigen had been added was divided into two portions, one of which was directly used as a whole blood sample, and the other of which was subjected to centrifugation to obtain a plasma sample. Each of the whole blood sample and the plasma sample was diluted three times before use. A concentration of the HBsAg in each of these diluted samples was set to 0.40 IU/mL (Low) or 40.00 IU/mL (Mid).

Each sample was introduced into passage 320 through liquid injection port 330 with a pipette tip, and fed to reciprocate therein (primary reaction). After removing the sample from passage 320 though liquid injection port 330, passage 320 was cleaned once with a cleaning solution. Subsequently, a labeling reagent (a mouse anti-HBsAg monoclonal antibody (Institute of Immunology Co., Ltd.)) labeled, via an amino group, with CF dye (Biotium, Inc.) was introduced into passage 320 through liquid injection port 330 and fed to reciprocate therein (secondary reaction). After removing the labeling reagent from passage 320 through liquid injection port 330, passage 320 was cleaned three times with a cleaning solution. Subsequently, a measurement liquid was introduced into passage 320 through liquid injection port 330. In this state, a fluorescent value was measured by SPFS. Specifically, metal film 120 was irradiated with excitation light (laser light) from the side of prism 110 with the incident angle of the excitation light against metal film 120 set to a reinforcement angle, and fluorescence emitted at this point was detected. A precedently measured optical blank value was subtracted from the thus obtained fluorescent value to calculate a signal value in correlation with the amount of the HBsAg. A precedently prepared calibration curve was used to calculate a concentration (IU/mL) (quantitative value) of the HBsAg based on the signal value. The concentration of the HBsAg in the whole blood sample was corrected by using a hematocrit value measured by a micro-hematocrit method.

The measurement results of the respective samples are shown in Table 1.

**[Table 1]**

| Blood No. | Concentration of Antigen Added | Quantitative Value (IU/mL) | | Proportion of Measured Value for Whole Blood relative to Measured Value for Plasma (%) |
|---|---|---|---|---|
| | | Plasma | Whole Blood | |
| 1 | Low | 0.43 | 0.46 | 107.3 |
| | Mid | 37.91 | 42.02 | 110.9 |
| 2 | Low | 0.46 | 0.44 | 97.4 |
| | Mid | 38.53 | 41.51 | 107.7 |
| 3 | Low | 0.34 | 0.33 | 97.0 |
| | Mid | 27.41 | 29.15 | 106.3 |
| 4 | Low | 0.38 | 0.38 | 99.0 |
| | Mid | 31.06 | 32.01 | 103.1 |

It is understood from Table 1 that a concentration of an HBsAg can be measured, even when whole blood is used as a specimen, in the same manner as in using plasma. The measured values for blood No. 3 or No. 4 were slightly lower than the concentration of the HBsAg added to the sample probably because of fluctuation caused in preparation of the sample, specimen characteristics or the like.

### Experiment 2: Confirmation of Daily Reproducibility

Detection chip 300 was prepared in the same manner as in Experiment 1. Four commercially available HBsAg-positive plasma (ProMedEx) samples were prepared as specimens. In the same manner as in Experiment 1, each specimen was measured for a signal value twice a day for 3 days to calculate a concentration (IU/mL) (quantitative value) of the HBsAg.

The measurement results of the respective specimens are shown in Table 2.

**[Table 2]**

| Blood No. | | Day 1 | | Day 2 | | Day 3 | | Average | Standard Deviation SD | Variation Coefficient CV (%) |
|---|---|---|---|---|---|---|---|---|---|---|
| | | First Time | Second Time | First Time | Second Time | First Time | Second Time | | | |
| 1 | Signal Value | 14540 | 13516 | 12402 | 13640 | 14228 | 14413 | 13790 | 795.6 | 5.8 |
| | Concentration (IU/mL) | 0.19 | 0.18 | 0.16 | 0.18 | 0.19 | 0.19 | 0.18 | 0.012 | 6.5 |
| 2 | Signal Value | 82484 | 80569 | 74866 | 73307 | 70451 | 74641 | 76053 | 4562.5 | 6.0 |
| | Concentration (IU/mL) | 1.34 | 1.31 | 1.20 | 1.18 | 1.12 | 1.20 | 1.22 | 0.082 | 6.7 |
| 3 | Signal Value | 208815 | 216284 | 195721 | 185472 | 180415 | 191192 | 196316 | 13798.8 | 7.0 |
| | Concentration (IU/mL) | 3.77 | 3.92 | 3.51 | 3.30 | 3.20 | 3.42 | 3.52 | 0.276 | 7.8 |
| 4 | Signal Value | 438207 | 429762 | 409178 | 408362 | 431070 | 442226 | 426467 | 14456.1 | 3.4 |
| | Concentration (IU/mL) | 8.60 | 8.41 | 7.96 | 7.95 | 8.44 | 8.68 | 8.34 | 0.314 | 3.8 |

It is understood from Table 2 that a variation coefficient CV is as low as 7.8% or less, and that the detection method for an HBsAg of the present embodiment has high daily reproducibility.

### Experiment 3: Confirmation of Dilution Linearity

Detection chip 300 was prepared in the same manner as in Experiment 1. Three commercially available HBsAg-positive plasma (ProMedEx) samples were prepared as specimens. Each plasma was diluted by once, 4 times, 16 times, 64 times, 256 times, 1024 times, 4096 times and 16384 times to prepare diluted samples. Each diluted sample was measured for a signal value to calculate a concentration (IU/mL) (quantitative value) of the HBsAg in the same manner as in Experiment 1.

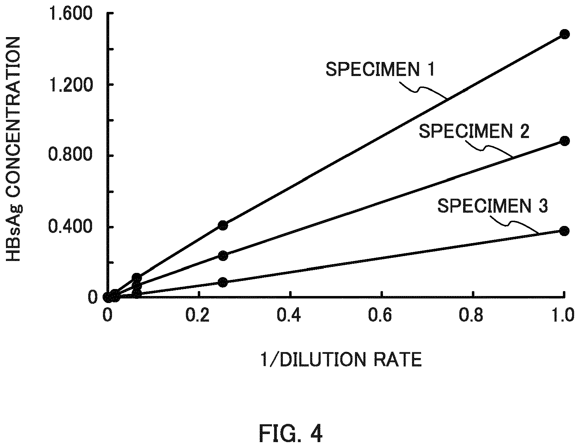

The measurement results of the respective specimens are illustrated in FIG. 4. It is understood from FIG. 4 that dilution linearity is good, and that detection can be performed at an arbitrary dilution rate by the detection method for an HBsAg of the present embodiment.

### Experiment 4: Comparison with Another Detection Method

Detection chip 300 was prepared in the same manner as in Experiment 1. Nineteen commercially available HBsAg-positive plasma (ProMedEx) samples were prepared as specimens. Each specimen was measured for a signal value to calculate a concentration (IU/mL) (quantitative value) of the HBsAg in the same manner as in Experiment 1.

In each of the same nineteen specimens, the HBsAg was detected by using a commercially available automatic chemiluminescent immunoassay apparatus (ADVIA Centaur; Siemens).

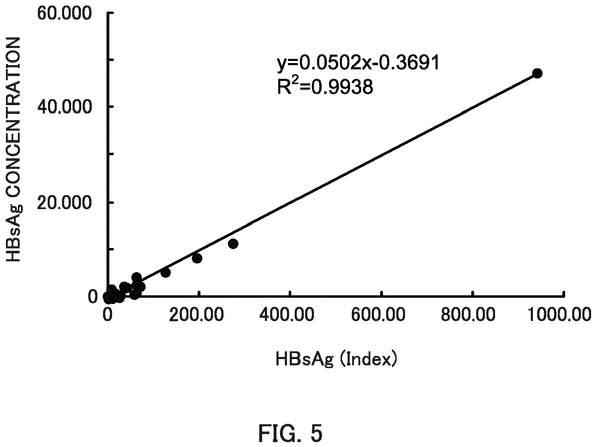

The measurement results of the respective specimens are illustrated in FIG. 5. The abscissa of a graph of FIG 5 corresponds to the measurement result (Index) obtained by the commercially available apparatus, and the ordinate corresponds to the measurement result (IU/mL) obtained by the detection method for an HBsAg of the present embodiment. It is understood from FIG. 5 that the detection method for an HBsAg of the present embodiment is highly correlated with the measurement result obtained by another measurement method, and that the measurement results are reliable. A cut-off value for determining HBsAg-positivity is 0.005 (IU/mL) in the detection method of the present embodiment, a cut-off value for determining HBsAg-positivity is 1.0 (Index) in the measurement method using the commercially available apparatus, and determination results for the positivity were the same in both the methods.

The present application is based upon and claims the benefit of priority of Japanese Patent Application No. 2017-167618, filed on August 31, 2017. The entire contents of the specification and drawings thereof are incorporated herein by reference.

### Industrial Applicability

When a detection method or a detection kit for an HBsAg of the present embodiment is employed, infection with HBV can be highly precisely examined in a short period of time. Accordingly, the detection method and the detection kit for an HBsAg of the present invention are useful for, for example, laboratory examinations and the like.

### Reference Signs List

- 100, 200, 300: detection chip
- 110: prism
- 111: entrance surface
- 112: film surface
- 113: exit surface
- 120: metal film
- 130: anti-hepatitis B virus surface antigen (HBsAg) antibody
- 140: hepatitis B virus surface antigen (HBsAg)
- 150: fluorescent substance
- 210: metal film
- 211: diffraction grating
- 310: passage cover
- 320: passage
- 330: liquid injection port
- 331: liquid injection port covering film
- 340: reservoir
- 341: reservoir covering film
- 342: vent
- 350: adhesive layer
- L1: excitation light
- L2: reflected light
- L3: fluorescence

## Claims

1. A detection method for a hepatitis B virus surface antigen, comprising:
preparing a detection chip including a metal film and a binding substance which is immobilized on the metal film and which specifically binds to a hepatitis B virus surface antigen;
providing a specimen onto the metal film to cause a hepatitis B virus surface antigen contained in the specimen to bind to the binding substance;
labeling, with a fluorescent substance, the hepatitis B virus surface antigen before or after binding to the binding substance; and
detecting fluorescence emitted from the fluorescent substance when the metal film is irradiated with excitation light in such a manner as to generate surface plasmon resonance in the metal film with the hepatitis B virus surface antigen labeled with the fluorescent substance kept in a state binding to the binding substance.

2. The detection method for a hepatitis B virus surface antigen according to claim 1, wherein the hepatitis B virus surface antigen is labeled with the fluorescent substance through binding to a binding substance specifically binding to a hepatitis B virus surface antigen and having been labeled with the fluorescent substance.

3. The detection method for a hepatitis B virus surface antigen according to claim 2, wherein the binding substance immobilized on the metal film and the binding substance labeled with the fluorescent substance are both anti-hepatitis B virus surface antigen antibodies.

4. The detection method for a hepatitis B virus surface antigen according to claim 3,
wherein the anti-hepatitis B virus surface antigen antibody immobilized on the metal film is one or two or more anti-hepatitis B virus surface antigen monoclonal antibodies or anti-hepatitis B virus surface antigen polyclonal antibodies, and
the anti-hepatitis B virus surface antigen antibody labeled with the fluorescent substance is one or two or more anti-hepatitis B virus surface antigen monoclonal antibodies or anti-hepatitis B virus surface antigen polyclonal antibodies different from the anti-hepatitis B virus surface antigen antibody immobilized on the metal film.

5. The detection method for a hepatitis B virus surface antigen according to claim 3, wherein the anti-hepatitis B virus surface antigen antibody immobilized on the metal film and the anti-hepatitis B virus surface antigen antibody labeled with the fluorescent substance are both antibodies binding to an S region of the hepatitis B virus surface antigen.

6. The detection method for a hepatitis B virus surface antigen according to claim 3, wherein the anti-hepatitis B virus surface antigen antibody immobilized on the metal film and the anti-hepatitis B virus surface antigen antibody labeled with the fluorescent substance both include both of an antibody binding to an S region of the hepatitis B virus surface antigen and an antibody binding to a Pre-S2 region of the hepatitis B virus surface antigen.

7. The detection method for a hepatitis B virus surface antigen according to any one of claims 3 to 6, wherein the fluorescent substance binds to the anti-hepatitis B virus surface antigen antibody via an amino group or a sulfhydryl group of the anti-hepatitis B virus surface antigen antibody.

8. The detection method for a hepatitis B virus surface antigen according to any one of claims 1 to 7, wherein the specimen is blood.

9. The detection method for a hepatitis B virus surface antigen according to any one of claims 1 to 8,
wherein the metal film is disposed on a prism, and
the metal film is irradiated with the excitation light through the prism.

10. The detection method for a hepatitis B virus surface antigen according to any one of claims 1 to 8,
wherein the metal film includes a diffraction grating,
the binding substance is immobilized on the diffraction grating, and
the diffraction grating is irradiated with the excitation light.

11. The detection method for a hepatitis B virus surface antigen according to any one of claims 1 to 10, wherein the excitation light is laser light emitted from a laser light source having an output power of 15 to 30 mW.

12. A detection kit for a hepatitis B virus surface antigen, comprising:
a detection chip including a metal film and a binding substance which is immobilized on the metal film and which specifically binds to a hepatitis B virus surface antigen; and
a labeling reagent for labeling a hepatitis B virus surface antigen with a fluorescent substance.

13. The detection kit for a hepatitis B virus surface antigen according to claim 12,
wherein the labeling reagent is a binding substance specifically binding to a hepatitis B virus surface antigen and having been labeled with a fluorescent substance.

14. The detection kit for a hepatitis B virus surface antigen according to claim 13, wherein the binding substance immobilized on the metal film and the binding substance labeled with the fluorescent substance are both anti-hepatitis B virus surface antigen antibodies.
